# EUROPEAN PATENT SPECIFICATION

(11) **EP 1 363 871 B1**
(45) Date of publication and mention of the grant of the patent: **19.04.2006**
(21) Application number: 01918356.5
(22) Date of filing: 28.02.2001
(51) Int. Cl.: C07C 68/06, C07C 69/96, C07C 31/20, C07C 27/02

(54) **PROCESS FOR CO-PRODUCTION OF DIALKYL CARBONATE AND ALKANEDIOL**
VERFAHREN ZUR GLEICHZEITIGEN HERSTELLUNG VON DIALKYLKARBONAT UND ALKANDIOL
PROCEDE DE COPRODUCTION DE CARBONATE DE DIALKYLE ET D'ALCANEDIOL

(43) Date of publication of application: 26.11.2003
(73) Proprietor: EXXONMOBIL OIL CORPORATION, Baytown, Texas 77520 (US)
(72) Inventor: JIANG, Zhaozhong, Edison, NJ 08817 (US); LAPIERRE, Rene, B., Guilford, CT 06437 (US); SANTIESTEBAN, Jose G., Baton Rouge, LA 70810 (US); TIMKEN, Hye Kyung Cho, Albany, California 96706 (US); WEBER, William A., Burlington, New Jersey 08016 (US)
(74) Representative: Dew, Melvyn John
(86) International application number: PCT/US2001/007038
(87) International publication number: WO 2002/070452

(56) References cited:
- EP-A- 0 478 073
- US-A- 5 430 170
- US-A- 6 166 240
- DATABASE WPI Section Ch, Week 9114 Derwent Publications Ltd., London, GB; Class A60, AN 1991-099158 XP002183057 & JP 03 044354 A (DAICEL CHEM IND), 26 February 1991 (1991-02-26) cited in the application
- DATABASE WPI Section Ch, Week 9420 Derwent Publications Ltd., London, GB; Class E19, AN 1994-163881 XP002183058 & JP 06 107601 A (NIPPON SHOKUBAI), 19 April 1994 (1994-04-19) cited in the application

## Description

### BACKGROUND

This invention relates to a method of co-producing an organic carbonate and an alkanediol, and, in particular, to a method for enhancing the efficiency of the co-production by the use of a IIIA metal oxide catalyst containing hydroxyl groups.

Various homogeneous catalysts have been proposed for carbonate transesterification. For example, U.S. Pat. Nos. 3,642,858 and 4,181,676 disclose the preparation of dialkyl carbonates by transesterifying alkylene carbonates with alcohols in the presence of alkali metals or alkali metal compounds without the use of a support material. U.S. Pat. No. 4,661,609 teaches the use of a catalyst selected from the group consisting of zirconium, titanium and tin oxides, salts or complexes thereof.

Commercial use of homogeneous catalysts is restricted because separation of the catalyst from the unconverted reactants and organic product can be difficult. Because the transesterification is an equilibrium reaction, in an attempt to isolate the intended carbonate by distillation of the reaction liquid without advance separation of the catalyst, the equilibrium is broken during the distillation and a reverse reaction is.induced. Thus, the carbonate once formed reverts to alkylene carbonate. Furthermore, due to the presence of the homogenous catalyst, side reactions such as decomposition, polymerization, or the like concurrently take place during the distillation which decrease the efficiency.

Various heterogenous catalysts have also been proposed for carbonate transesterification. The use of alkaline earth metal halides is disclosed in U.S. Pat. No. 5,498,743. Knifton, et al., "Ethylene Glycol-Dimethyl Carbonate Cogeneration," *J. Molec. Catal.* 67:389-399 (1991) disclose the use of free organic phosphines or organic phosphines supported on partially crosslinked polystyrene. U.S. Pat. No. 4,691,041 discloses the use of organic ion exchange resins, alkali and alkaline earth silicates impregnated into silica, and certain ammonium exchanged zeolites. U.S. Pat. No. 5,430,170 discloses the use of a catalyst containing a rare earth metal oxide as the catalytically active component. The use of hydrotalcites is disclosed in Japanese Unexamined Patent Application 3[1991]-44,354. The use of MgO is disclosed in Japanese Unexamined Patent Application 6[1994]-107,601. The use of zeolites ion-exchanged with alkali metal and/or alkaline earth metal, thereby containing a stoichiometric amount of metal, are disclosed in U.S. Pat. No. 5,436,362. EP-A-478 073 discloses the preparation of carboxylated compounds such as dialkylcarbonate and ethyleneglycol using a mixed metal oxide catalyst or a modified bimetallic or polymetallic catalyst.

Inorganic heterogenous catalysts generally possess thermal stability and easy regeneration. However, these catalysts, including the zeolites containing a stoichiometric amount of alkali or alkaline earth metal, generally demonstrate low activity and/or selectivity and are unsatisfactory for commercial application.

Polymer supported organic phosphines and ion exchange resins show high activity and good to excellent selectivity in transesterification reaction between alkylene carbonate and alkanol; however, these polymeric materials do not appear very stable and gradually lose catalytic activity, especially at relatively high temperatures.

Thus, there remains a need for a method of transesterifying alkylene carbonate with alkanol to co-produce dialkyl carbonate and alkanediol which will provide higher feed conversion and product selectivity over a wide temperature range

### SUMMARY OF INVENTION

A method is provided for co-producing an organic carbonate and an alkanediol by reacting an alkylene carbonate with an alkanol or a phenolic compound under transesterification process conditions in the presence of a IIIA metal oxide catalyst which is not a mixed metal oxide. The preferred alkylene carbonate is ethylene carbonate and the preferred alkanol is methanol.

The preferred metal oxide of the catalyst is alumina. It is also preferred that the catalyst contain hydroxyl groups. The hydrated catalyst will usually contain hydroxyl groups in an amount greater than 0.1 wt% and less than 30 wt% of the catalyst. Another preferred embodiment includes the catalyst having an octahedral structure. The catalyst can further include an inert catalyst support.

The alumina catalyst is preferably prepared through dehydration of aluminum trihydroxide or aluminum monohydroxide, such as pseudoboehmite.

The process conditions include a reaction temperature of about 20°C (68°F) to about 300°C (572°F), a reaction pressure of about 96 kPag to 27.58 mPag (about 14 to about 4000 psig), a liquid hour space velocity of about 0.1 to 40 hr⁻¹, and a molar ratio of alkanol to alkylene carbonate of about 1-20.

Unlike polymer catalysts such as ion exchange resins, the IIIA metal oxide catalysts used in the method of the invention are thermally stable and regenerable. The combination of high catalytic activity and selectivity in a wide temperature range, and excellent thermal stability and regenerability of the catalysts, render them suitable for commercial use in co-producing organic carbonate and alkanediol through ester exchange reaction. Also, the general availability and low cost of alumina catalysts could significantly improve the process economics.

The organic carbonates produced by the method of the invention, dimethyl carbonate in particular, have potential application as "green" replacements for phosgene that is used mainly in manufacture of polyurethane and polycarbonate resins.

### BRIEF DESCRIPTION OF DRAWINGS

Figure 1 is a graph demonstrating EC (ethylene carbonate) conversion vs. temperature for the MeOH/EC reaction using pseudoboehmite alumina, γ-alumina, and Cs-alumina as the catalysts.

Figure 2 is a graph demonstrating DMC (dimethyl carbonate) selectivity vs. temperature for the MeOH/EC reaction using pseudoboehmite alumina, γ-alumina, and Cs-alumina as the catalysts.

Figure 3 is a graph demonstrating EG (ethylene glycol) selectivity vs. temperature for the MeOH/EC reaction using pseudoboehmite alumina, γ-alumina, and Cs-alumina as the catalysts.

### DETAILED DESCRIPTION OF INVENTION

In accordance with the present invention, a method is provided for the co-production of organic carbonate and alkanediol through the transesterification of alkylene carbonate with alkanol or phenolic compound using a IIIA metal oxide catalyst which is not a mixed metal oxide.

Generally, all alkylene carbonates can be used as a reactant in this invention. However, low molecular weight alkylene carbonate such as ethylene carbonate, propylene carbonate, butylene carbonate or the like is preferred; ethylene carbonate or propylene carbonate is most preferred.

Generally, all alkanol reactants can be used, provided the alkanol reacts with cyclocarbonate to produce the dialkyl carbonate and alkanediol product. However, an aliphatic or aromatic alkanol having 1 to 10 carbon atoms is preferably used. For example, methanol, ethanol, n-propanol, iso-propanol, n-butanol, iso-butanol, secondary butanol, tertiary butanol, allyl alcohol, pentanol, cyclo-hexanol, benzyl alcohol, 2-phenyl ethyl alcohol, 3-phenyl propyl alcohol, 2-methoxy ethanol or the like can be used as the aliphatic or aromatic alcohol. A lower aliphatic alcohol such as methanol is most preferably used due to its reactivity and low cost.

Further, a phenolic compound can be used in place of the alcoholic compound as the compound which has a hydroxyl (OH) group and reacts with cyclocarbonate to produce the carbonate.

The catalyst used in the method of the invention is a IIIA metal oxide catalyst. The metal is chosen from the IIIA metals listed on the Periodic Table of elements. Aluminum, gallium, and indium are preferred IIIA metals. Aluminum is most preferred, making alumina the preferred oxide. The catalyst used in the method of the invention will contain only a single metal and, therefore, will not contain a mixed metal oxide. The IIIA metal oxide is the active component and can be supported on any known catalyst support.

In a preferred embodiment, the catalyst used in the method of the invention contains hydroxyl groups. Hydroxyl group content is defined by the weight of hydroxyl group versus the total weight of the catalyst. A large portion of these hydroxyl groups generally reside on the surface of the catalyst. The hydrated catalyst will usually contain hydroxyl groups in an amount greater than 0.1 wt% and less than about 30 wt% of the catalyst; preferably between about 0.3 and 15 wt% of the catalyst. It is believed that the presence of the hydroxyl groups contributes to a higher activity of the catalyst.

Most of the commercial catalysts of the type used in the method of the invention, such as γ-alumina, have a surface area of about 200-300 m²/g. A high surface area is preferred in the method of the invention, with no specific upper limit, The catalysts used in the method of the invention typically have a surface area of between about 5-600 m²/g. A surface area above 50 m²/g is preferred.

The IIIA metal oxide catalyst will generally have octahedral or a mixture of octahedral and tetrahedral configuration. Tetrahedral configuration means that the IIIA metal, such as aluminum, is coordinated with four oxygen atoms and forms a tetrahedral geometric structure. Octahedral configuration means that the IIIA metal is coordinated with six oxygen atoms, thereby forming an octahedral structure. Penta-coordinated oxides do exist. However, they are mainly formed during a transition from octahedral to tetrahedral configuration, such as when an oxide material is being dehydrated. The penta-coordinated configuration is usually present in a relatively small quantity, if at all, in the final IIIA metal oxide catalyst.

It is preferred that the catalyst used in the method of the invention contain a metal, e.g. aluminum, in octahedral form. Potential catalysts and catalyst precursors that can be utilized in the method of the invention are aluminum trihydroxides such as Gibbsite, Bayerite, and Nordstrandite which can be described as a chemical formula, Al₂O₃.3H₂O; and aluminum mono-hydroxides such as Boehmite, Pseudoboehmite, Diaspore which can be described as a chemical formula, Al₂O₃.H₂O. These materials are also favored because they contain a high hydroxyl content. Among aluminum mono-hydroxides, Pseudoboehmite (fine boehmite) is desirable for its high surface area and excellent retention of surface area upon calcination. In addition, transient alumina with a high surface area is appropriate for this application, which includes alumina in Gamma, Eta, Delta, Chi, and Theta forms. The nomenclature, structure and transformation of alumina phases have been published by W. H. Gitzen, "Alumina As a Ceramic Material," American Ceramic Society, Inc, Columbus, OH, (1970); J. W. Newsome, et al., "Alumina Properties, Technical Paper No. 10," Alcoa Chemical Company of America, Pittsburgh, PA, (1960).

The IIIA metal oxide catalyst can also contain alkali metal, alkaline earth metal, or a combination thereof. Alkali metal and alkaline earth metal are both defined to include compounds containing these metals. The specific limits of the amount of alkali and/or alkaline earth metal which can be incorporated into the catalyst can be determined by one skilled in the art, and will vary based upon the specific catalyst and alkali and/or alkaline earth metal used. The amount of alkali and/or alkaline earth metal in the catalyst should not exceed an amount where the pore space of the catalyst is significantly restricted, thereby decreasing the surface area of the catalyst and its activity. For example, if cesium is used as the alkali metal, the amount of cesium will typically be between about 3-50 wt% of the catalyst. Alkali and/or alkaline earth metal may be incorporated into the alumina catalyst by any known means, such as incipient wetness impregnation method.

The reactor type in this invention can be any type generally known, such as a continuous fluid bed, fixed bed or stirred tank, etc. With the heterogenous catalyst used in the method of the invention, it is preferred that a fixed bed be used so as to avoid the expense of having to recover the catalyst from the reagents and product.

The reaction conditions of this invention include a reaction temperature of about 20°C to about 300°C, preferably about 60°C to about 175°C; a reaction pressure of about 96 kPag to about 27.58 mPag (about 14 to about 4000 psig), preferably about 345 kPag to about 2.76 MPag about 50 to about 400 psig); a liquid hour space velocity of about 0.1 to about 40 hr⁻¹, preferably about 0.5 to about 10 hr⁻¹; and a molar ratio of alkanol to alkylene carbonate of about 1 to 20, preferably about 2 to 8.

The following comparative examples are provided to assist in a further understanding of the invention. The particular materials and conditions employed are intended to be further illustrative of the invention and are not limiting upon the reasonable scope thereof

### EXAMPLE 1

This example describes a method for preparing three catalysts employed in the examples.

Three catalysts employed were: pseudoboehmite (fine particle boehmite) alumina, γ-alumina (prepared from the pseudoboehmite alumina via calcination at 538°C), and Cs-containing alumina. Boehmite is a form of alumina with molecular formula (AlO-OH)ₙ. The physical properties of the three alumina samples are shown in Table 1.

**TABLE 1**

| Catalyst | BET Surface Area (m²/g) | H Contentₐ (millimole/g) | OH, wt%_{b} |
|---|---|---|---|
| Pseudoboehmite alumina | 356 | 3.7 | 6.3 |
| γ-alumina | 263 | 1.8 | 3.1 |
| Cs-alumina | 101 | 1.2 | 2.0 |

The pseudoboehmite alumina, a commercial sample, was pelleted and sized to 80-120 mesh prior to catalyst evaluation.

The γ-alumina was prepared by mulling the commercial pseudoboehmite alumina powder with water to form uniform extrudable mixture and formed into 0.32 cm (1/8") cylindrical shape extrudates using a standard augur extruder. The extrudates were dried on a belt filter at 250°F (121°C) and calcined under air at 1000°F (538°C) for 3 hours. X-ray powder diffraction showed the phase was converted to the gamma form. The extrudate sample was sized to 80-120 mesh prior to catalyst evaluation.

The Cs-alumina catalyst was prepared from the above pseudoboehmite extrudate after drying. Dried alumina extrudates were impregnated with 22% Cs using a solution containing cesium carbonate via incipient wetness impregnation method. The Cs impregnated alumina extrudates were dried at 250°F (121°C) overnight and calcined under air at 1000°F (538°C) for 3 hours. The calcined Cs-alumina sample contained 22.2 wt% Cs and was sized to 80-120 mesh prior to catalyst evaluation.

### EXAMPLE 2

This example describes the NMR characterization of the structures and H content of three alumina catalysts; pseudoboehmite, γ-alumina, and Cs-alumina.

500.13 MHZ proton MAS NMR spectra were obtained on a Bruker AMX spectrometer using 12-15 kHz sample spinning, 2.7 µs pulses, and a 30s recycle time. Chemical shifts are referenced to external TMS at 0.0 ppm. The samples were prepared for proton NMR experiments by drying at 100°C (to remove physisorbed water) under vacuum for 12 hours. An 83.8/16.2 mixture of D₂O (99% deuterated) and H₂O was used as the absolute ¹H quantitation standard. The results are summarized in Table 1.

130.33 MHZ ²⁷Al MAS NMR spectra were obtained on a 500 MHZ Bruker AMX spectrometer using 4.0-4.5 kHz sample spinning, 0.8 µs excitation pulses, an 80 ms recycle time, and high power proton decoupling. 1M Al(NO₃)₃ was the external chemical shift reference.

The pseudoboehmite catalyst is an alumina hydrate containing exclusively octahedral aluminum (surrounded by six oxygen) and high concentration of hydroxyl groups. γ-alumina, on the other hand, contains tetrahedral and penta-coordinated aluminum in addition to octahedral aluminum, and low concentration of hydroxyl groups.

The ²⁷Al NMR spectrum of the pseudoboehmite showed a single peak at 5.4 ppm due to octahedral aluminum while three ²⁷Al resonance peaks at 5.4, 28.0, and 62.1 ppm corresponding to octahedral, penta-coordinated, and tetrahedral aluminum, respectively, were observed for γ-alumina. Cs-alumina exhibited two absorbtions at 5.4 and 62.1 ppm due to corresponding octahedral and tetrahedral aluminum.

### EXAMPLE 3

Transesterification evaluations were performed using each of the catalysts described in Example 1.

The transesterification evaluations were performed in a fixed bed microunit equipped with a three-zone furnace and a down-flow trickle-bed tubular reactor of 1.27 cm (½") ID. Catalyst powder was pelletized and sized to 80-120 mesh, and the reactor was loaded with 10 cc of the sized catalyst.

After pressure testing of the unit, the catalyst was dried at 204°C (400°F) for two hours under 101.3 kPa (1 atmosphere), 170 cc/min nitrogen flow. At the end of this period, the reactor was cooled down to 150°F (66°C) and nitrogen flow was stopped. The reactor pressure, controlled by a pressure regulator, was then set to 690 kPa (100 psi), and the EC/methanol mixture feed was pumped and added to the top of the reactor at 1.0 h⁻¹ LHSV. The reactor temperature was gradually increased to initial operating temperature (250°F) (121°C), Each material balance was typically started after the reactor was conditioned for eight hours. Liquid products were condensed in a stainless steel dropout pot at -10°C. Both liquid and off-gas products were analyzed by GC. The catalytic reaction was studied at various temperatures and LHSV to vary EC conversion.

The three alumina catalysts were evaluated according to the procedures described above. Detailed operating conditions, material balance data, and results on EC conversion and dimethyl carbonate (DMC)/ethylene glycol (EG) selectivities for pseudoboehmite alumina, γ-alumina, and Cs-alumina, are summarized in Tables 2, 3, and 4, respectively.

Feed conversion is calculated based on EC converted during the transesterification reaction since an excessive amount of methanol (relative to EC) was used for all reactions. During EC/MeOH reaction, 2-hydroxyethyl methyl carbonate (HEMC) intermediate was also formed in addition to DMC and EG. The concentration of HEMC varies depending on the reaction conditions. Since it is recyclable along with unreacted EC, the intermediate carbonate is not considered as a byproduct. The feed conversion and product selectivity are defined as follows:
EC Conversion = (EC converted to products other than HEMC)/(total EC in feed)
DMC Selectivity = (moles of DMC formed)/(moles of EC converted to products other than HEMC)
EG Selectivity = (moles of EG formed)/(moles of EC converted to products other than HEMC).

**TABLE 2 Transesterification of Ethylene Carbonate with Methanol Catalyzed by Pseudoboehmite Alumina (Condition: 690 kPag (100 psig))**

| Temperature, °F/°C | 250/121 | 275/135 | 300/149 | 325/163 | 350/177 | 325/163 | 325/163 | 275/135 |
|---|---|---|---|---|---|---|---|---|
| LHSV, h⁻¹ | 1.0 | 1.0 | 1.0 | 1.0 | 1.0 | 4.0 | 6.0 | 0.5 |
| | | | | | | | | |
| Feed Composition | | | | | | | | |
| MeOH/EC, molar ratio | 4.0 | 4.0 | 4.0 | 4.0 | 4.0 | 4.0 | 4.0 | 4.0 |
| | | | | | | | | |
| Total Liquid Product Composition | | | | | | | | |
| MeOH, wt% | 47.8 | 44.8 | 43.3 | 46.5 | 53.9 | 46.1 | 47.5 | 42.9 |
| EC, wt% | 18.8 | 16.5 | 14.8 | 16.2 | 12.0 | 18.8 | 20.2 | 13.9 |
| HEMC Intermediate, wt% | 10.0 | 7.3 | 5.9 | 3.0 | 1.7 | 4.9 | 5.3 | 6.9 |
| DMC, wt% | 15.0 | 19.1 | 22.0 | 19.1 | 18.4 | 18.2 | 16.6 | 22.6 |
| EG, wt% | 10.4 | 13.3 | 14.9 | 11.7 | 7.2 | 12.4 | 11.4 | 15.6 |
| DMC/EG, Molar Ratio | 1.00 | 0.99 | 1.02 | 1.13 | 1.76 | 1.01 | 1.00 | 1.00 |
| | | | | | | | | |
| EC Conv., % | 36.0 | 46.4 | 53.2 | 55.5 | 69.1 | 44.8 | 40.8 | 54.0 |
| DMC Selec., % | 100.0 | 98.9 | 99.1 | 81.5 | 60.8 | 98.1 | 98.0 | 99.3 |
| EG Select., % | 100.0 | 99.8 | 97.3 | 72.3 | 34.5 | 96.8 | 97.6 | 99.3 |

**TABLE 3 Transesterification of Ethylene Carbonate with Methanol Catalyzed by γ-Alumina (Condition: 690 kPag (100 psig))**

| Temperature, °F/°C | 250/121 | 275/135 | 300/149 | 325/163 | 350/177 | 325/163 | 325/135 |
|---|---|---|---|---|---|---|---|
| LHSV, h⁻¹ | 1.0 | 1.0 | 1.0 | 1.0 | 1.0 | 4.0 | 6.0 |
| | | | | | | | |
| Feed Composition | | | | | | | |
| MeOH/EC, molar ratio | 4.0 | 4.0 | 4.0 | 4.0 | 4.0 | 4.0 | 4.0 |
| | | | | | | | |
| Total Liquid Product Composition | | | | | | | |
| MeOH, wt% | 50.9 | 49.6 | 48.0 | 52.5 | 57.3 | 51.0 | 52.5 |
| EC, wt% | 25.1 | 22.8 | 19.9 | 20.7 | 13.7 | 23.4 | 25.7 |
| HEMC Intertnediate, wt% | 8.8 | 6.9 | 3.8 | 1.6 | 0.9 | 2.8 | 3.1 |
| DMC, wt% | 8.7 | 12.3 | 16.6 | 12.2 | 12.5 | 12.8 | 10.5 |
| EG, wt% | 6.6 | 8.3 | 10.4 | 5.3 | 3.3 | 8.3 | 7.0 |
| DMC/EG, Molar Ratio | 0.91 | 1.62 | 1.10 | 1.59 | 2.61 | 1.06 | 1.04 |
| | | | | | | | |
| EC Conv., % | 22.8 | 30.4 | 43.8 | 47.4 | 67.2 | 35.9 | 29.4 |
| DMC Selec., % | 91.2 | 99.1 | 91.9 | 60.6 | 41.6 | 88.0 | 88.1 |
| EG Select., % | 100.0 | 96.9 | 83.4 | 38.1 | 15.9 | 82.7 | 85.1 |

**TABLE 4 Transesterification of Ethylene Carbonate with Methanol Catalyzed by Cs-Alumina (Condition: 690 kPag (100 psig)**

| Temperature, °F/°C | 250/121 | 275/135 | 300/149 | 325/163 | 350/177 | 325/163 | 325/135 |
|---|---|---|---|---|---|---|---|
| LHSV, h⁻¹ | 1.0 | 1.0 | 1.0 | 1.0 | 1.0 | 4.0 | 6.0 |
| | | | | | | | |
| Feed Composition | | | | | | | |
| MeOH/EC, molar ratio | 4.0 | 4.0 | 4.0 | 4.0 | 4.0 | 4.0 | 4.0 |
| | | | | | | | |
| Total Liquid Product Composition | | | | | | | |
| MeOH, wt% | 51.5 | 49.4 | 46.9 | 48.2 | 60.1 | 51.0 | 52.1 |
| EC, wt% | 24.5 | 21.8 | 18.9 | 18.0 | 17.4 | 24.1 | 25.7 |
| HEMC Intermediate, wt% | 9.6 | 7.0 | 4.2 | 2.5 | 1.0 | 3.4 | 3.6 |
| DMC, wt% | 8.3 | 12.8 | 17.6 | 17.4 | 9.0 | 12.4 | 10.5 |
| EG, wt% | 6.1 | 8.8 | 11.5 | 10.4 | 2.4 | 8.2 | 7.0 |
| DMC/EG, Molar Ratio | 0.94 | 1.00 | 1.06 | 1.15 | 2.59 | 1.04 | 1.04 |
| | | | | | | | |
| EC Conv., % | 22.1 | 32.6 | 45.8 | 52.0 | 58.4 | 33.9 | 28.7 |
| DMC Selec., % | 90.7 | 96.1 | 92.9 | 79.3 | 34.6 | 88.8 | 90.1 |
| EG Select., % | 96.6 | 95.7 | 87.9 | 68.7 | 13.4 | 85.1 | 87.1 |

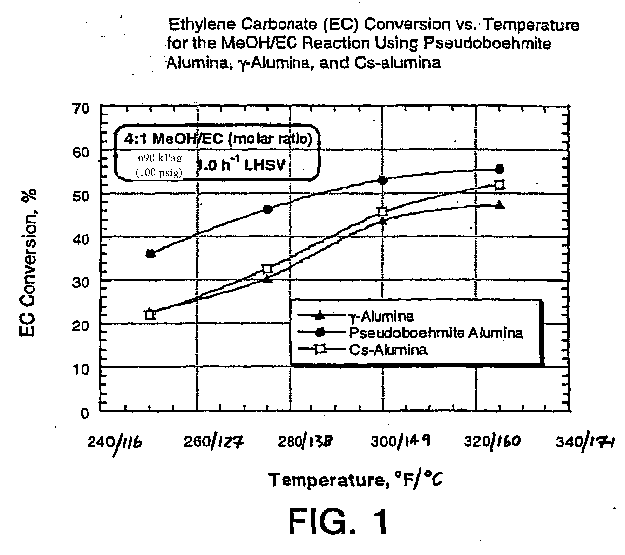

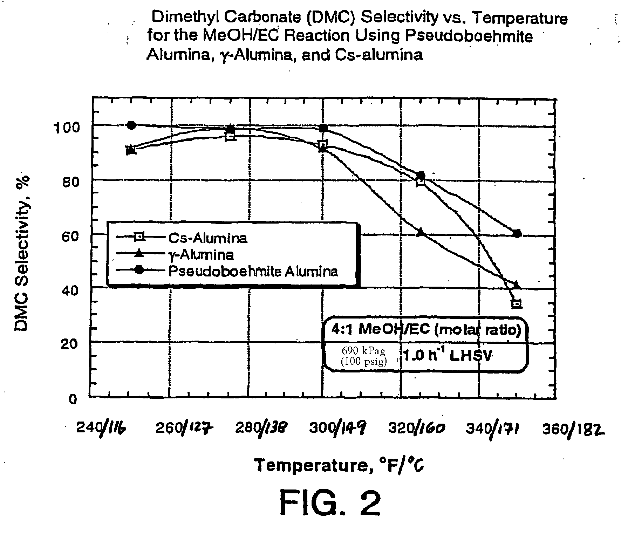

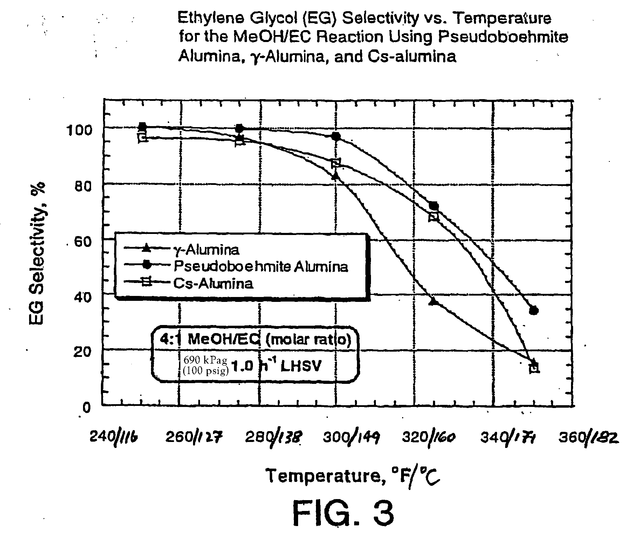

The comparisons among the three catalysts are made under similar process conditions (feed ratio, pressure, LHSV, etc) and are shown in Figures 1-3. The results demonstrate that a good EC conversion is obtainable with a very high DMC/EG selectivity. Further, the high DMC/EG selectivity is observed over a wider temperature range than for conventional catalysts. An increase in EC conversion is observed with an increase in temperature.

Table 2 in particular, which relates to the performance of pseudoboehmite, shows that good EC conversion, and ≥98% DMC and EG selectivity are obtainable under optimal reaction conditions. Pseudoboehmite alumina (containing a larger amount of surface hydroxyl groups) exhibits higher activity and DMC/EG selectivity over a wide temperature range vs. γ-alumina and Cs-alumina. Apparently, hydroxyl groups play an important role in promoting the catalytic activity. The observed high selectivity of the hydrated alumina could be due to its low acidity, which inhibits side reactions such as dehydration of alkanols and subsequent hydrolysis of organic carbonates.

Therefore, the method of the invention is adaptable to commercial application because of the good level of activity, very high selectivity over a wide temperature range, and the stability and relatively low cost of the IIIA metal oxide catalyst used.

## Claims

1. A method for co-producing an organic carbonate and an alkanediol comprising reacting an alkylene carbonate with an alkanol or a phenolic compound under transesterification process conditions in the presence of a IIIA metal oxide catalyst which is not a mixed metal oxide.

2. The method of Claim 1 wherein the alkylene carbonate is ethylene carbonate and wherein the alkanol is methanol.

3. The method of Claim 1 or 2 wherein the IIIA metal oxide is alumina.

4. The method of Claim 3 wherein the alumina is aluminum trihydroxide or aluminum monohydroxide.

5. The method of Claim 4 wherein the aluminum monohydroxide is pseudoboehmite.

6. The method of any preceding Claim wherein at least a portion of the IIIA metal oxide catalyst has an octahedral structure.

7. The method of any preceding Claim wherein the IIIA metal oxide contains hydroxyl groups.

8. The method of Claim 7 wherein the hydroxyl groups are present in an amount greater than 0.1 wt% and less than 30 wt% of the catalyst.

9. The method of any preceding Claim wherein the catalyst further comprises an inert catalyst support.

10. The method of any preceding Claim wherein the process conditions comprise a reaction temperature of 20°C to 300°C, a reaction pressure of 96 kPag to 27.58 MPag (14 to 4000 psig), a liquid hour space velocity of 0.1 to 40 hr⁻¹, and a molar ratio of alkanol to alkylene carbonate of 1-20.

## Patentansprüche

1. Verfahren zur Coproduktion eines organischen Carbonats und eines Alkandiols, bei dem ein Alkylencarbonat mit einem Alkanol oder einer phenolischen Verbindung unter Umesterungsverfahrensbedingungen in Gegenwart eines IIIA-Metalloxid-Katalysators umgesetzt wird, der kein gemischtes Metalloxid ist.

2. Verfahren nach Anspruch 1, bei dem das Alkylencarbonat Ethylencarbonat ist und das Alkanol Methanol ist.

3. Verfahren nach Anspruch 1 oder 2, bei dem das IIIA-Metalloxid Aluminiumoxid ist.

4. Verfahren nach Anspruch 3, bei dem das Aluminiumoxid Aluminiumtrihydroxid oder Aluminiummonohydroxid ist.

5. Verfahren nach Anspruch 4, bei dem das Aluminiummonohydroxid Pseudoböhmit ist.

6. Verfahren nach einem der vorhergehenden Ansprüche, bei dem mindestens ein Teil des IIIA-Metalloxid-Katalysators eine oktaedrische Struktur hat.

7. Verfahren nach einem der vorhergehenden Ansprüche, bei dem das IIIA-Metalloxid Hydroxylgruppen enthält.

8. Verfahren nach Anspruch 7, bei dem die Hydroxylgruppen in einer Menge von mehr als 0,1 Gew.-% und weniger als 30 Gew.-% des Katalysators vorhanden sind.

9. Verfahren nach einem der vorhergehenden Ansprüche, bei dem der Katalysator ferner einen inerten Katalysatorträger umfasst.

10. Verfahren nach einem der vorhergehenden Ansprüche, bei dem die Verfahrensbedingungen eine Reaktionstemperatur von 20°C bis 300°C, einen Reaktionsdruck von 96 kPa Überdruck bis 27,58 MPa Überdruck (14 bis 4000 psig), einen stündlichen Flüssigkeitsdurchsatz von 0,1 bis 40 h⁻¹ und ein Molverhältnis von Alkanol zu Alkylencarbonat von 1 bis 20 umfassen.

## Revendications

1. Procédé pour la coproduction d'un carbonate organique et d'un alcanediol, comprenant la réaction d'un carbonate d'alkylène avec un alcanol ou un composé phénolique dans des conditions de procédé de transestérification en présence d'un catalyseur à base d'oxyde de métal du Groupe IIIA, qui n'est pas un oxyde métallique mixte.

2. Procédé suivant la revendication 1, dans lequel le carbonate d'alkylène est le carbonate d'éthylène, et dans lequel l'alcanol est le méthanol.

3. Procédé suivant la revendication 1 ou 2, dans lequel l'oxyde de métal du Groupe III est l'alumine.

4. Procédé suivant la revendication 3, dans lequel l'alumine est le trihydroxyde d'aluminium ou le monohydroxyde d'aluminium.

5. Procédé suivant la revendication 4, dans lequel le monohydroxyde d'aluminium est la pseudoboehmite.

6. Procédé suivant l'une quelconque des revendications précédentes, dans lequel au moins une partie du catalyseur à base d'oxyde de métal du Groupe IIIA possède une structure octaédrique.

7. Procédé suivant l'une quelconque des revendications précédentes, dans lequel l'oxyde de métal du Groupe IIIA contient des groupes hydroxyle.

8. Procédé suivant la revendication 7, dans lequel les groupes hydroxyle sont présents en une quantité supérieure à 0,1 % en poids et inférieure à 30 % du catalyseur.

9. Procédé suivant l'une quelconque des revendications précédentes, dans lequel le catalyseur comprend en outre un support inerte de catalyseur.

10. Procédé suivant l'une quelconque des revendications précédentes, dans lequel les conditions du procédé comprennent une température réactionnelle de 20°C à 300°C, une pression de réaction de 96 kPag à 27,58 Mpag (14 à 4000 psig), une vitesse spatiale horaire de liquide de 0,1 à 40 h⁻¹ et un rapport molaire de l'alcanol au carbonate d'alkylène de 1 à 20.
